# EUROPÄISCHE PATENTANMELDUNG

(11) **EP 2 067 763 A2**
(43) Veröffentlichungstag der Anmeldung: **10.06.2009**
(21) Anmeldenummer: 08169333.5
(22) Anmeldetag: 18.11.2008
(51) Int. Cl.: C07C 45/64, C07C 49/513

(54) **Verfahren zur Herstellung von substituierten Decalin-6-ol-1-on-Derivaten**

(30) Priorität: 08.12.2007 DE 102007059214
(71) Anmelder: Saltigo GmbH, 40764 Langenfeld (DE)
(72) Erfinder: Baskakov, Denys, 51379, Leverkusen (DE); Job, Andreas, 50858, Köln (DE); Gless, Peter, 51371, Leverkusen (DE); Rodefeld, Lars, 51375, Leverkusen (DE)
(74) Vertreter: Pettrich, Klaus-Günter

(57) **Zusammenfassung**

Beschrieben wird ein Verfahren zur Herstellung von Verbindungen der Formel: 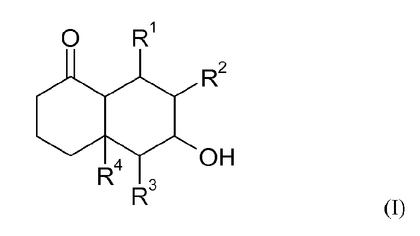 in der R¹, R², R³, R⁴ unabhängig von einander für Wasserstoff, C₁-C₆-Alkyl oder Aryl stehen, dadurch gekennzeichnet, dass die Verbindungen der Formel (II): 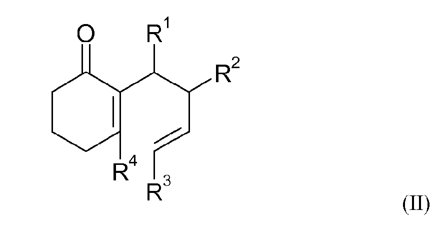 in der R¹, R², R³, R⁴ vorstehend genannte Bedeutung besitzen in Gegenwart eines Katalysators in einer Lösung von 1 bis 50 Gew.-% an Verbindungen der Formel (II) cyclisiert werden und das dabei entstandene Reaktionsgemisch gegebenenfalls destillativ aufgearbeitet wird.

Ebenfalls beansprucht wird deren Verwendung in einem Verfahren zur Herstellung von Agrochemikalien, pharmazeutischen Wirkstoffen, Riech- und Aromastoffen, Geschmacksstoffen, Kosmetika und Polymeren.

## Beschreibung

Die vorliegende Erfindung betrifft ein Verfahren zur Herstellung von substituierten Decalin-6-ol-1-on-Derivaten durch Cyclisierung einer Vorstufe, die in einer bestimmten Konzentration vorliegt, in Gegenwart eines Katalysators.

Decalin-6-ol-1-on-Derivate sind wertvolle Ausgangsprodukte für die Herstellung von Agrochemikalien, pharmazeutischen Wirkstoffen oder Wirkstoffen im kosmetischen Bereich.

Die Synthese von substituierten Decalinderivaten ist durch eine Vielzahl von Reaktionen in der Literatur beschrieben. Diels-Alder Reaktion ermöglicht die Synthese von verschiedenen DecalinDerivaten. Allerdings liefert Diels-Alder keine befriedigenden und technisch anwendbaren Lösungen für die Synthese von Decalinderivaten welcher an der 10-Position substituiert sind. Birch-Reduktion von 6-Methoxy-1-tetralol mit der anschließenden Oppenhauer Oxidation liefert ein Enolether-Keton welches durch die Umsetzung mit Dimethyllithiumkuprat in ein 10-Methyldecalinderivat umgewandelt werden kann. Dieser Syntheseweg besitzt den Nachteil der hohen Stufenzahl und der notwendigen chromatographischen Reinigung der Zwischenstufen. Weiterhin müssen bei der Durchführung von Birchreduktion stöchiometriche Mengen von einer Natrium Lösung in flüssigem Ammoniak eingesetzt werden, was technisch nicht praktikabel ist.

Aus Tetrahedron Letters 1977, 38, 3321 ist weiterhin bekannt, dass substituierte Decaline auch durch kationische Cyclisierungen in Gegenwart von einem Überschuss an Perchlorsäure in einer stark verdünnten Lösung zugänglich sind. In technischem Maßstab ist die Handhabung von großen Mengen Perchlorsäure mit hohem sicherheitstechnischem Risiko verbunden und sollte daher vermieden werden. Die starke Verdünnung der Reaktionslösung ist für die intramolekularen Ringschlüsse von ganz entscheidender Bedeutung, wie in K. Peter, C. Vollhardt, Neil E. Schore, Organische Chemie, 4. Auflage, 2005, Wiley-VCH Verlag, Weinheim, Seiten 56-57 und 403 beschrieben ist. Derartige Reaktionsführungen sind aber großtechnisch sehr nachteilig, da die RaumZeitausbeute stark verringert wird und der erforderliche Einsatz von großen Lösungsmittel Mengen das Verfahren unpraktikabel machen. Die Notwendigkeit der säulenchromatographischen Aufarbeitung von Reaktionsgemischen erschwert den großtechnischen Einsatz von diesem Verfahren in ganz entscheidender Weise.

Wegen der großen Bedeutung von Decalinderivaten bestand daher das Bedürfnis, ein Verfahren zu deren Herstellung bereitzustellen, das die oben geschilderten Nachteile vermeidet.

Gegenstand der vorliegenden Erfindung ist ein Verfahren zur Herstellung von Verbindungen der Formel (I) 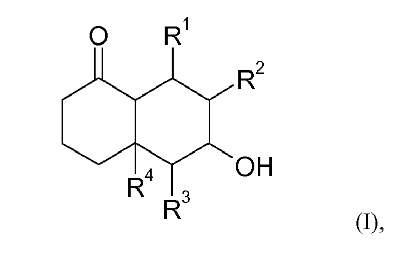 in der R¹, R², R³, R⁴ unabhängig von einander für Wasserstoff, C₁-C₆-Alkyl oder Aryl stehen, dadurch gekennzeichnet, dass Verbindungen der Formel (II): 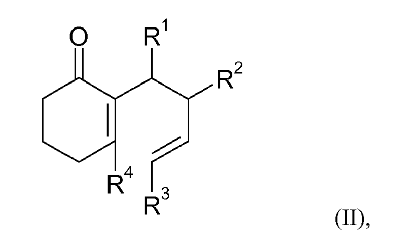 in der R¹, R², R³, R⁴ vorstehend genannten Bedeutung besitzen, in Gegenwart eines Katalysators in einer Lösung enthaltend 1,0 bis 50 Gew.-% an Verbindungen der Formel (II), cyclisiert werden und das dabei entstandene Reaktionsgemisch gegebenenfalls destillativ aufgearbeitet wird.

Als Alkyl für R¹, R², R³, R⁴ kommen C₁-C₆-Alkyl oder falls R¹, R², R³, R⁴ für Aryl steht, Phenyl für Aryl in Frage.

Besonders bevorzugt steht R¹, R², R³ für Wasserstoff und R⁴ für Methyl.

Das erfindungsgemäße Verfahren wird in Gegenwart eines Katalysators durchgeführt. Katalysatoren sind beispielsweise Brönstedt-, Lewis- und konjugierte Brönstedt-Lewissäuren. Bevorzugte Brönstedtsäuren sind solche Verbindungen, Ionen oder Gruppen, für die die Hammetsche Aciditätsfunktion kleiner als -12, bevorzugt -12 bis -20, besonders bevorzugt -12 bis -15 und ganz besonders bevorzugt -12 bis -13 ist. Als Beispiele seien genannt: Perchlorsäure, Halogensulfonsäuren wie insbesondere Chlorsulfonsäure, Fluorsulfonsäure, Perfluoralkansulfonsäuren wie insbesondere Trifluormethansulfonsäure.

Bevorzugte Lewis-Säuren sind Halogenide, bevorzugt Fluoride oder Chloride von Bor, Aluminium, Gallium, Antimon, Zinn, Tantal, Titan, Zink, Arsen, Niob und anderen Übergangsmetallen. Vorgenannte Verbindungen können dabei auch in komplexierter Form, beispielsweise als deren Ether-, Alkohol oder Aminaddukte oder auch als Addukte mit korrespondierenden Halogenwasserstoffsäuren eingesetzt werden.

Bevorzugte konjugierte Brönstedt-Lewissäuren sind protische starke Säuren deren Acidität durch die Kombination mit Lewissäuren erhöht ist. Als Beispiele seien genannt oxygenierte Brönstedtsäuren wie Schwefelsäure, Fluorsulfonsäure, Perfluoralkansulfonsäuren wie insbesondere Trifluormethansulfonsäure in Kombination mit Lewissäuren wie Schwefeltrioxid, Fluoride oder Chloride von Bor, Aluminium, Gallium, Antimon, Zinn, Tantal, Titan, Zink, Arsen, Niob und anderen Übergangsmetallen.

Der Katalysator wird im erfindungsgemäßen Verfahren beispielsweise in einer Menge von 0.001 bis 50 mol-%, bezogen auf das Edukt der Formel (II) eingesetzt, bevorzugt 0.001 bis 25 mol-%, besonders bevorzugt 0.5 bis 10 mol%.

Bevorzugt wird als Katalysator Perchlorsäure, z.B. in einer Konzentration von 1 bis 10 mol-%, bezogen auf Edukt, eingesetzt.

Die Reaktionstemperatur für das erfindungsgemäße Verfahren kann beispielsweise zwischen -100 bis 200°C, vorzugsweise zwischen -20 bis 100°C liegen. Besonders bevorzugt wird die Reaktion bei Raumtemperatur (25°C) durchgeführt.

Der Reaktionsdruck für das erfindungsgemäße Verfahren kann beispielsweise von 1hPa bis 20MPa betragen, vorzugsweise 100hPa bis 2MPa und besonders bevorzugt ist der Reaktionsdruck der Umgebungsdruck.

Das erfindungsgemäße Verfahren kann in einem Lösungsmittel durchgeführt werden. Als Lösungsmittel kommen beispielsweise solche in Betracht, die in der Lage sind, Carbokatione abzufangen und in stabile Intermediate zu überführen, wie Wasser, Carbonsäureanhydride wie Essigsäureanhydrid, Maleinsäureanhydrid, Buttersäureanhydrid und Propionsäureanhydrid, Carbonsäurechloride, Carbonsäuren wie Essigsäure, Ameisensäure, Buttersäure und Propionsäure, Alkanole, Amine, aromatische Alkohole, Aldehyde und Ketone. Die Durchführung der Reaktion ist auch in einem Lösungsmittelgemisch möglich. Dabei kommen beispielsweise Mischungen aus einem vorher genannten reaktiven Lösungsmittel mit einem inerten Lösungsmittel wie Estern von Carbonsäuren wie Essigester, Butylacetat, Benzylbenzoat, aromatischen und/oder aliphatischen gegebenenfalls halogeniertem Kohlenwasserstoffen wie beispielsweise Benzin, Benzol, Toluol, Xylol, Chlorbenzol, Dichlorbenzol, Petrolether, Pentan, Hexan, Cyclohexan, Dichlormethan, Chloroform oder Tetrachlorkohlenstoff; Ether wie Diethylether, Diisopropylether, Dioxan, Methyltertbutylether, Tetrahydrofuran oder Ethylenglykoldimethyl- oder -diethyl ether; Nitrile, wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäureamid; Schwefelverbindungen wie Sulfolan zum Einsatz. Bevorzugte Lösungsmittel sind Carbonsäureanhydride und deren Mischungen mit Carbonsäuren, besonders bevorzugt ist Essigsäureanhydrid.

Es ist auch möglich die Reaktion ohne Zusatz von Lösungsmittel durchzuführen. Das gilt insbesondere dann, wenn das Edukt der Formel (II) unter Reaktionsbedingungen flüssig ist.

Bevorzugte Eduktkonzentrationen in der Reaktionslösung liegen zwischen 1 Gew..-% und 50 Gew.-%, besonders bevorzugt 5-20 Gew.-%, ganz besonders bevorzugt 8-15 Gew.-%.

Zur Durchführung der Reaktion kann man beispielsweise so vorgehen, dass die Verbindung der Formel (II) gegebenenfalls unter Zusatz von Lösungsmittel vorgelegt wird und der Katalysator oder gegebenenfalls seiner Lösung in einem Lösungsmittel zugegeben wird. Alternativ ist auch die Vorlage von Katalysator gegebenenfalls in einem Lösungsmittel und die anschließende Zugabe Edukt mit oder ohne Lösungsmittel möglich. Ebenso ist die Simultandosierung von Edukten und Katalysator möglich.

Die Aufarbeitung der Reaktionsgemische erfolgt durch die Neutralisation des Katalysators mit einer Base wie zum Beispiel einem Hydroxid, Carbonat und Hydrogencarbonat eines Metalls wie zum Beispiel Natrium, Kalium, Calcium, Lithium oder Cäsium. Bevorzugt sind Hydroxide von Alkalimetallen, besonders bevorzugt sind Kaliumhydroxid und Natriumhydroxid.

Die erhaltene Rohlösung kann im Vakuum zwischen 0.1 Pa und 100 kPa zur Aufreinigung destilliert werden. Bevorzugt liegt der Druckbereich zwischen 1 Pa und 50 kPa, besonders bevorzugt zwischen 1 Pa und 1 Pa.

Auf erfindungsgemäße Weise werden in einem einzigen Schritt in hoher chemischer Ausbeute nach einer einfachen Aufarbeitung die gewünschten Decalin-6-ol-1-on Derivate erhalten.

Die erfindungsgemäß herstellbaren Verbindungen der Formel (I) eignen sich insbesondere zur Anwendung in einem Verfahren zur Herstellung von Agrochemikalien, pharmazeutischen Wirkstoffen, Riech- und Aromastoffen, Geschmacksstoffen, Wirkstoffen im kosmetischen Bereich, und in den Polymeren.

### Beispiel

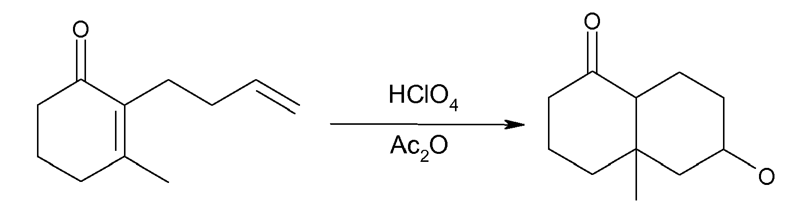

Unter starkem Rühren wurde zu Essigsäureanhydrid (250 ml) 70% Perchlorsäurelösung in Wasser (0.32 g, 3 mol%) zugesetzt. Danach wurde das Edukt (14.8 g) in Essigsäureanhydrid (100 ml) tropfenweise unter Rühren zugegeben. Nach 4 Stunden bei Raumtemperatur war kein Edukt in der Reaktionsmischung mehr nachweisbar. Die Reaktionsmischung wurde auf 200 ml IN NaOH gegossen und danach mit 45% NaOH auf pH 7-8 gestellt und mit Methyl-tert-Butylether (2 mal je 120 ml) extrahiert. Die vereinigten organischen Phasen wurden mit 2 mal je 40 ml dest. Wasser extrahiert, und über Na2SO4 getrocknet. Anschließend wurde das Lösungsmittel im Vakuum abdestilliert. Der verbleibende ölige Rückstand wurde in 10%iges methanolischen NaOH (200 ml) 1 Stunde gekocht. Die Reaktionsmischung wurde mit 200 ml Wasser verdünnt und mit DCM (2 mal je 200 ml) extrahiert. Die vereinigten organischen Phasen wurden mit 2 mal je 100 ml Wasser extrahiert und das Lösungsmittel der organischen Phase wurde im Vakuum entfernt. Vakuumdestillation bei 125 °C und 1 Pa ergibt das Produkt in Form eines klaren, leicht gelben Öls.

## Patentansprüche

1. Verfahren zur Herstellung von Verbindungen der Formel (I): 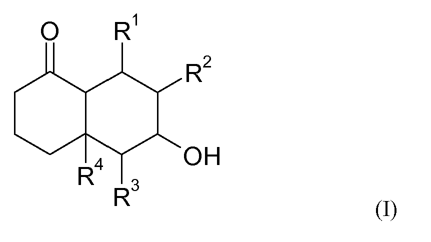 in der R¹, R², R³, R⁴ unabhängig von einander für Wasserstoff, C₁-C₆-Alkyl oder Aryl stehen, **dadurch gekennzeichnet, dass** Verbindungen der Formel (II): 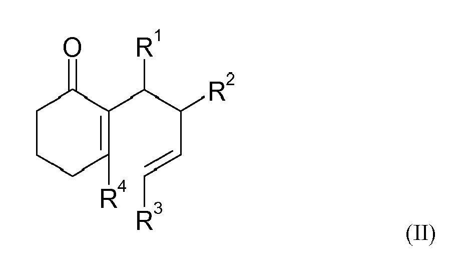 in der R¹, R², R³, R⁴ vorstehend genannte Bedeutung besitzen, in Gegenwart eines Katalysators in einer Lösung, enthaltend 1 bis 50 Gew.-% an Verbindungen der Formel (II), cyclisiert werden und das dabei entstandene Reaktionsgemisch gegebenenfalls destillativ aufgearbeitet wird.

2. Verfahren nach Anspruch 1 **dadurch gekennzeichnet, dass** der Katalysator eine Brönstedt-, Lewis- und/oder konjugierte Brönstedt-Lewissäure ist.

3. Verfahren nach Anspruch 2 **dadurch gekennzeichnet, dass** der Katalysator eine Brönstedtsäure, die nach der Hammetschen Aciditätsfunktion einen Wert kleiner als -12 aufweist, ist.

4. Verfahren nach Anspruch 2, **dadurch gekennzeichnet, dass** der Katalysator eine Lewissäure aus der Gruppe Halogenide von Bor, Aluminium, Gallium, Antimon, Zinn, Tantal, Titan, Zink, Arsen oder Niob als Halogenid selber oder dessen Komplex als Ether-, Alkohol-, Aminaddukt oder Addukt der korrespondierenden Halogenwasserstoffsäure ist.

5. Verfahren nach einem der Ansprüche 1 bis 4, **dadurch gekennzeichnet, dass** der Katalysator in einer Menge von 0.001 bis 50 Gew.-% bezogen auf die Verbindung der Formel (II), eingesetzt wird.

6. Verfahren nach einem der Ansprüche 1 bis 5, **dadurch gekennzeichnet, dass** die Reaktion in einer Lösung von 5 bis 20 Gew.-% an Verbindungen der Formel (II) durchgeführt wird.

7. Verfahren nach einem der Ansprüche 1 bis 6, **dadurch gekennzeichnet, dass** die destillative Aufarbeitung des Reaktionsgemisches durch eine Vakuumdestillation zwischen 0.1 Pa und 100 kPa durchgeführt wird.

8. Verfahren nach einem der vorherigen Ansprüche 1 bis 7, **dadurch gekennzeichnet, dass** der Katalysator in einem Carbonsäureanhydrid als Lösungsmittel vorgelegt und die Verbindungen der Formel (II) tröpfchenweise zudosiert werden.

9. Verwendung von Verbindungen der Formel (I), hergestellt nach einem der Ansprüche 1 bis 8, in einem Verfahren zur Herstellung von Agrochemikalien, pharmazeutischen Wirkstoffen, Riech- und Aromastoffen, Geschmacksstoffen, Kosmetika und Polymeren.
